# EUROPEAN PATENT SPECIFICATION

(11) **EP 1 061 070 B1**
(45) Date of publication and mention of the grant of the patent: **16.07.2008**
(21) Application number: 99905280.6
(22) Date of filing: 23.02.1999
(51) Int. Cl.: C07C 401/00, C07C 35/32, C07C 49/443, C07C 43/178, C07C 33/44, C07F 7/18, A61K 31/59

(54) **Process for the preparation of ED-71**
Verfahren zur Herstellung von ED-71
Procédé pour la préparation de ED-71

(30) Priority: 24.02.1998 JP 4229598
(43) Date of publication of application: 20.12.2000
(73) Proprietor: CHUGAI SEIYAKU KABUSHIKI KAISHA, Tokyo, 115-8543 (JP)
(72) Inventor: HATAKEYAMA, Susumi, Nagasaki-shi Nagasaki 852-8027 (JP); WATANABE, Hiroyoshi, Gotenba-shi Shizuoka 412-8513 (JP); KAWASE, Akira, Gotenba-shi Shizuoka 412-8513 (JP)
(74) Representative: Vossius & Partner
(86) International application number: PCT/JP1999/000796
(87) International publication number: WO 1999/043645

(56) References cited:
- EP-A- 0 184 206
- EP-A- 1 072 582
- JP-A- 8 301 811
- JP-A- 9 500 117
- JP-A- 51 108 048
- JP-A- 55 022 655
- JP-A- 58 208 223
- JP-A- 61 267 549
- JP-A- 63 107 929
- OSTREM V K ET AL: "THE VITAMIN D-INDUCED DIFFERENTIATION OF HL-60 CELLS: STRUCTURAL REQUIREMENTS" STEROIDS, BUTTERWORTH-HEINEMANN, STONEHAM, MA, US, vol. 49, no. 1-3, January 1987 (1987-01), pages 73-102, XP009014554 ISSN: 0039-128X
- KONNO K ET AL: "A novel and practical route to A-ring enyne synthon for 1alpha,25-dihydroxyvitamin D3 analogs: synthesis of A-ring diastereomers of 1alpha,25-dihydroxyvitamin D3 and 2-methyl-1,25-dihydroxyvitamin D3" BIOORGANIC & MEDICINAL CHEMISTRY LETTERS, OXFORD, GB, vol. 8, no. 2, 20 January 1998 (1998-01-20), pages 151-156, XP004136835 ISSN: 0960-894X
- HATAKEYAMA S ET AL: "Convergent synthesis of 1alpha,25-dihydroxy-2beta-(3-hydroxypropox y)v itamin D3 (ED-71)" BIOORGANIC & MEDICINAL CHEMISTRY LETTERS, OXFORD, GB, vol. 7, no. 22, 18 November 1997 (1997-11-18), pages 2871-2874, XP004136547 ISSN: 0960-894X
- JOONGGON KIM et al., "A Facile Synthetic Route to an A-Ring Trihydroxylated Vitamin D Analog from D-Arabinose", BULL. KOREAN CHEM. SOC., 1997, Vol. 18, No. 5, pages 459-461, XP002929210
- J. MAEYAMA ET AL: "Two convergent approaches to the sytnehsis of 1.alpha.25-dihydroxy-2.beta.-(3-hydroxypro poxy)-Vitamin D3" HETEROCYCLES., vol. 70, 2006, pages 295-307, NLELSEVIER SCIENCE PUBLISHERS B.V. AMSTERDAM.
- I. HIJIKURO ET AL: "Parallel Synthesis of Vitamin D3 Lirary in the Solid Phase" JOURNAL OF THE AMERICAN CHEMICAL SOCIETY., vol. 123, 2001, pages 3716-3722, USAMERICAN CHEMICAL SOCIETY, WASHINGTON, DC.

## Description

The present invention relates to a process for preparing vitamin D derivatives of formula (6).

Recently, some of the physiological activities of vitamins D have been revealed. It is known that a certain vitamin D, for example, 1α,25-dihydroxyvitamin D₃, exhibits a variety of physiological activities such as a calcium metabolism-controlling activity, a proliferation-inhibiting activity and a differentiation-inducing activity on cells such as tumor cells, and an immune-controlling activity.

However, 1α,25-dihydroxyvitamin D₃ disadvantageously causes hypercalcemia depending on the dose and/or the administration route and thus is not suitable for use as an antitumor agent, a therapeutic agent for rheumatic diseases, etc. In order to isolate such activities of the vitamin D derivatives, numerous vitamin D derivatives have been synthesized these days and their physiological activities were evaluated.

Among numerous vitamin D₃ derivatives, some of those which have a substituent at the 2β-position possess physiological activities such as calcium metabolism-controlling activity and differentiation-inducing activity on cells such as tumor cells and are known to be useful as a medicine, such as a therapeutic agent for diseases associated with abnormal calcium metabolism, such as osteoporosis, osteomalacia, etc. and an antitumor agent (Japanese Patent Publication (Kokoku) No. 3-14303, Japanese Patent Publication (Kokai) No. 61-267549 and Japanese Patent Publication (Kokai) No. 6-41059). Among them, 2β-(3-hydroxypropoxy)-1α,25-dihydroxyvitamin D₃ is expected to be of practical use in treating osteoporosis, with a high blood level being able to be maintained for a long duration.

Vitamin D₃ derivatives may be synthesized, for example, by epoxidation and then opening the A ring structure of a steroid compound (used as a starting material) so as to introduce the substituent to 2-position (Japanese Patent Publication (Kokai) No. 61-267549) or by coupling the A ring part and the CD ring part of the vitamin D derivative, which parts have been synthesized separately. Japanese Patent Publication (Kokai) No. 6-25039 and Japanese Patent Application No. 9-53316 (published as Japanese Patent Publication (Kokai) No. 10-251183) disclose processes for synthesizing the A ring part of vitamin D derivatives.

As described above, the 2β-(3-hydroxypropoxy)-1α,25-dihydroxyvitamin D₃ has been developed as a useful medicine; at the same time, its metabolites are also under study. With recent progress in the study of 2β-(3-hydroxypropoxy)-1α,25-dihydroxyvitamin D₃ metabolites, it has been suggested that some of the metabolites have their side chain hydroxylated.

The synthesis of these compounds is disclosed in Bioorg. Med. Chem. Lett. vol. 7(22) pp 2871-2874 (1997).

The object of the present invention is to provide a process for synthesizing 2β-(3-hydroxypropoxy)-1α,25-dihydroxyvitamin D₃.

A process corresponding to that of the present invention, is a process for preparing vitamin D derivatives having the general formula (1): 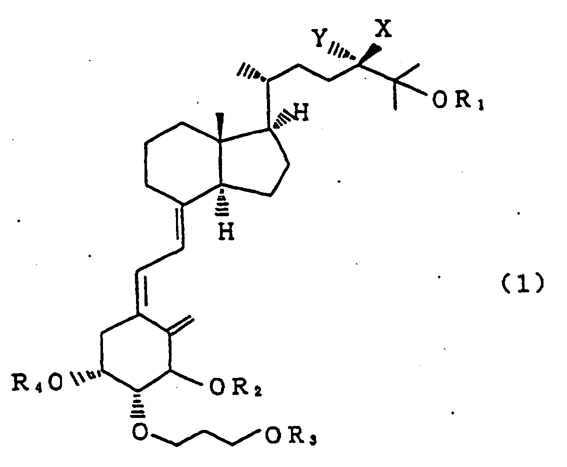 [wherein R₁, R₂, R₃ and R₄, which may be the same or different, represent a hydrogen atom or a protecting group; X and Y represent H or OR₅, provided Y is OR₅ when X is H and Y is H when X is OR₅ (wherein R₅ is a hydrogen atom or a protecting group and R₁ and R₅ may together form a vicinal-diol protecting group)] comprising:
reacting a compound having the general formula (4): 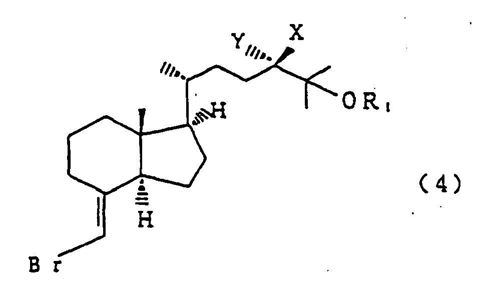 [wherein R₁ represents a hydrogen atom or a protecting group; X and Y represent H or OR₅, provided Y is OR₅ when X is H and Y is H when X is OR₅ (wherein R₅ represents a hydrogen atom or a protecting group and R₁ and R₅ may together form a vicinal-diol protecting group)]
with a compound of the general formula (5): 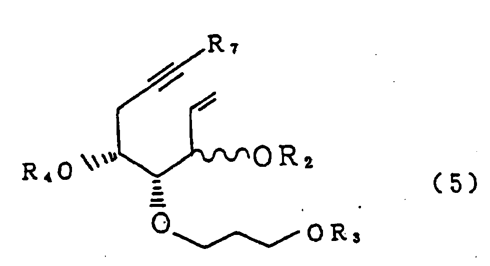
(wherein R₇ represents a hydrogen atom; and R₂, R₃ and R₄, which are the same or different, represent a hydrogen atom or a protecting group).

In the above process, the OR₂ group at the 3-position of the compound having the general formula (5) is in R-configuration and the 1-position of the compound having the general formula (1) is in R-configuration.

According to the present invention, there is provided a process for preparing vitamin D derivatives having the general formula (6): 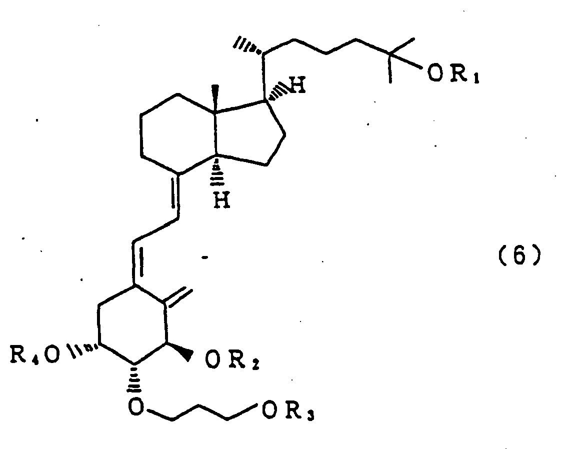 (wherein R₁, R₂, R₃ and R₄, which may be the same or different, represent a hydrogen atom or a protecting group) comprising:
reacting a compound having the general formula (7): 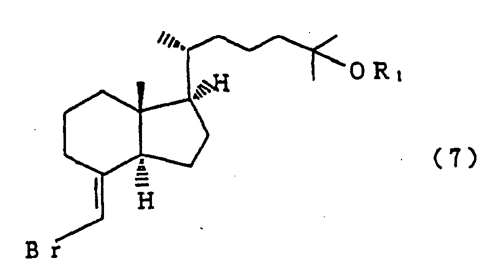 (wherein R₁ represents a hydrogen atom or a protecting group)
with a compound having the general formula (5): 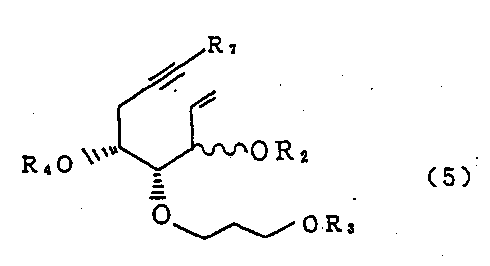 (wherein R₇ represents a hydrogen atom; and R₂, R₃ and R₄, which are the same or different, represent a hydrogen atom or a protecting group).

The following are detailed embodiments of the present invention and specific methods for carrying it out.

As used herein, R₁ to R₄ each represent a hydrogen atom or a protecting group. "Protecting group" refers to a hydroxy group protecting group and includes any protecting groups removable by conventional deprotecting means (e.g. hydrolysis, oxidative cleavage, reductive cleavage and hydrogenlysis) substantially free of harmful effects on any other portions of the molecule.

Non-limiting examples of the protecting groups are as follows:
(I) an acyl group represented by R^{a}CO- (wherein R^{a} is a hydrogen atom, a C1-C6 alkyl group, a C1-C6 haloalkyl group or an aryl group); for example, formyl, acetyl, propionyl, butyryl, isobutyryl, valeryl, isovaleryl, pivaloyl, caproyl, benzoyl, trifluoroacetyl, etc.
(II) an alkoxycarbonyl group represented by R^{b}OCO- (wherein R^{b} is a C1-C6 alkyl group, a C1-C6 alkenyl group, a C7-C9 aralkyl group, or an aryl group); for example, methoxycarbonyl, ethoxycarbonyl, propoxycarbonyl, isopropoxycarbonyl, allyloxycarbonyl, benzyloxycarbonyl, phenoxycarbonyl, etc.
(III) a trisubstituted silyl group represented by the following formula: 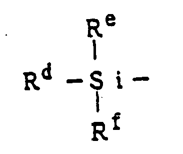 (wherein R^{d}, R^{e} and R^{f}, which are the same or different, represent a C1-C6 alkyl group, an aryl group or a C7-C9 aralkyl group); for example, trimethylsilyl, triethylsilyl, triisopropylsilyl, t-butyldimethylsilyl, t-butyldiphenylsilyl, tribenzylsilyl, etc.
(IV) a 1-alkoxy or 1-mercaptoalkyl group represented by the following formula: 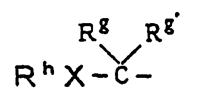 (wherein X represents an oxygen atom or a sulfur atom and R^{g} and R^{g'} each represent a hydrogen atom or a C1-C6 alkyl group; R^{h} is a substituted or unsubstituted C1-C6 alkyl group [examples of the substituent are a lower alkoxy group, a halogen atom such as chlorine, an alkyl substituted silyl group (e.g. trimethylsilyl group) and a phenyl group which may be substituted with an alkoxy group, a halogen atom, etc.]}; for example, methoxymethyl, methoxyethoxymethyl, 1-ethoxyethyl, methoxyisopropyl, methylthiomethyl, t-butylthiomethyl, β-trichloroethyloxymethyl, trimethylsilylethoxymethyl, p-methoxybenzyloxymethyl, p-chlorobenzyloxymethyl, etc.
(V) a 2-oxacycloalkyl group represented by the following formula: 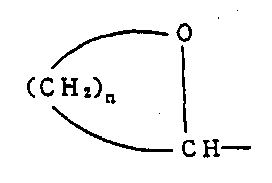 (wherein n is an integer of 3 to 6); for example, a tetrahydrofuralyl group, a tetrahydropyranyl group, etc.
(VI) an aralkyl group such as a benzyl group:

As the A ring part, the compounds of the general formula (5) are used.

The compound of the general formula (5) may be synthesized as typically shown in Example 21 to be described later: first, acetylene is added to 1,2-epoxy-4-pivaloyloxy-3-(3'-pivaloyloxypropoxy)-5-hexene which is mentioned in Japanese Patent Application No. 9-53316 (published as Japanese Patent Publication (Kokai) No. 10-251183), and then appropriately adding and/or removing and/or substituting with a protecting group. The reaction of acetylene addition can be carried out as follows: a compound having a metal-acetylene-protecting group structure is produced by adding a solution of a metallic alkyl (e.g. n-butyl lithium) in solvent to a solution of acetylene having a protecting group (e.g. trimethylsilyl group) in an inert solvent (e.g. tetrahydrofuran (THF)) solution and then a solution of 1,2-epoxy-4-pivaloyloxy-3-(3'-pivaloyloxypropoxy)-5-hexene in solvent is added thereto. The reaction temperature can appropriately be selected according to the type of the solvent and other factors; generally, -78°C to 0°C, preferably, -78°C to -50°C. The reaction time can also be selected appropriately; generally, 1 to 5 hours, preferably, 2 to 3 hours.

The compound of the general formula (5) and compound of the general formula (4), which correspond to the A ring part and the CD ring part of the compounds of formula (1), respectively, are coupled, for example, under the conditions described in Example 22 to give a vitamin D derivative having an optionally protected hydroxy group at 24-position.

The coupling reaction may be carried out by subjecting the above-mentioned compounds to reaction in a solvent such as THF, acetonitrile, benzene, toluene, xylene, dimethylsulfoxide, N,N-dimethylformamide, N,N-dimethylacetamide, etc. These solvents may be used either alone or in admixture. Toluene is preferred among these solvents. Although not being particularly limited, the reaction temperature is generally at 25 to 140°C, preferably at 60 to 110°C. Although not being particularly limited, the reaction time ranges generally from 2 to 12 hours, preferably from 3 to 6 hours.

Active vitamin D derivatives can be obtained by removing the hydroxy group protecting group, after the coupling reaction as required. Such addition and removal of protecting groups are well known to those skilled in the art.

According to the present invention, there is provided a process by which 2β-(3-hydroxypropoxy)-1α,25-dihydroxyvitamin D₃ (this compound per se is known) which possesses useful physiological activities is synthesized by using the compound of the general formula (5) corresponding to the A ring part of the vitamin D derivatives. Specifically, the desired product can be obtained by coupling the compound of the general formula (5) with the compound of the general formula (7). Conditions for the coupling reaction are similar to those described for the coupling reaction between the compound of the general formula (5) and the compound of the general formula (4) and these can be appropriately selected by those skilled in the art.

The present invention will now be illustrated in greater detail with reference to the following Examples, but it should be understood that the present invention is by no means limited thereto.

Examples designated "Illustrative" serve to illustrate anologous processes not covered by the claim.

### Examples

### Illustrative Example 1: Synthesis of [3a(R)]-4(S)-acetyloxy-7a(R)-methyl-1(R)-[1(S)-methyl-2-phenylsulfonylethyl]-octahydro-1H-indene (compound 5)

Under cooling with ice, acetic anhydride (188 µl, 1.99 mmol) was added to a solution of [3a(R)]-4(S)-hydroxy-7a(R)-methyl-1(R)-[1(S)-methyl-2-phenylsulfonylethyl]-octahydro-1H-indene (compound 4; 334 mg, 994 µmol), pyridine (200 µl) and N,N-dimethylaminopyridine (DMAP) (18 mg) in dichloromethane (20 ml), followed by stirring for 16 hours at room temperature under argon atmosphere. The reaction mixture was poured into diluted hydrochloric acid, extracted with dichloromethane and washed with a saturated solution of sodium hydrogencarbonate (NaHCO₃). The organic layer was dried over magnesium sulfate (MgSO₄) and distilled to remove the solvent under reduced pressure; the resulting residue was purified by flash column chromatography (40% ethyl acetate/hexane) to give the titled compound (368 mg, 98%) as a colorless oil.
¹H NMR: δ 0.86(3H,s), 1.20(3H, d, J=6.8Hz), 2.03(3H, s), 2.84(1H, dd, J=14.1, 9.8Hz), 3.11(1H, d, J=14.IHz), 5,12(1H, brs), 7.52-7.96(5H, m);
IR (neat) cm⁻¹: 2945, 1735, 1310, 1250, 1150;
MS(m/z): 336 (M⁺-Ac), 135 (100%);
UV λ max nm: 270, 263, 257, 217.

### Illustrative Example 2: Synthesis of [3a(R)]-1(R)-[4(R),5-dihydroxy-1(S),5-dimethyl-2-phenylsulfonylhexyl]-4(S)-hydroxy-7a(R)-methyl-octahydro-1H-indene (compound 7a)

Under argon atmosphere at -20°C, n-butyllithium (1.63 M, 2.3 ml, 3.75 mmol) was added dropwise to a solution of [3a(R)]-4(S)-acetyloxy-7a(R)-methyl-1(R)-[1(S)-methyl-2-phenylsulfonylethyl]-octahydro-1H-indene (160 mg, 423 µmol) obtained in Example 1 and 2(R),3-dihydroxy-3-methyl-1-p-toluenesulfonyloxybutane (R. Dumont and H. Pfander, Helvetica Chimica Acta, 66, 814 (1983) and references cited therein) (232 mg, 847 µmol) in THF (15 ml), followed by stirring for 2 hours at the same temperature. The reaction mixture was poured into a saturated aqueous solution of ammonium chloride, extracted twice with ethyl acetate and washed with saturated aqueous sodium chloride. The organic layer was dried over magnesium sulfate (MgSO₄) and distilled under reduced pressure to remove the solvent; the resulting residue was purified by flash column chromatography (ethanol:dichloromethane = 0.3:5) to give the titled compound (131 mg, 71%) as colorless foam.
¹H NMR: δ 0.67(3H,s), 0.96(3H, d, J=6.8Hz), 1.20(3H,s), 1.26(3H,s), 3.51(1H, d, J=10.2Hz), 3.81(1H, brd, J=11.7Hz), 4.00(1H, brs), 7.52-7.94(5H,m);
IR (neat) cm⁻¹: 3500(br), 2935, 1300, 1280, 1135;
MS(m/z): 439(M⁺+1), 71(100%);
UV λ max nm: 271, 263, 257, 216.

### Illustrative Example 3: Synthesis of [3a(R)]-1(R)-[4(S),5-dihydroxy-1(S),5-dimethyl-2-phenylsulfonylhexyl]-4(S)-hydroxy-7a(R)-methyl-octahydro-1H-indene (compound 7b)

The same procedure as in Example 2 was repeated using [3a(R)]-4(S)-hydroxy-7a(R)-methyl-1(R)-[1(S)-methyl-2-phenylsulfonylethyl]-octahydro-1H-indene (compound 4; 305 mg, 908 µmol), 2(S),3-dihydroxy-3-methyl-1-paratoluenesulfonyloxybutane (R. Dumont and H. Pfander, Helvetica Chimica Acta, 66, 814 (1983) and references cited therein) (compound 6b; 249 mg, 909 µmol), THF (20 ml) and n-butyl lithium (1.69 M, 4.3 ml, 7.27 mmol), except that a mixture of ethyl acetate:hexane = 4:1 was employed as the solvent for column chromatography. The titled compound was obtained as a colorless oil (110 mg, 28%).
¹H NMR: δ 0.69(3H,s), 1.05(3H, d, J=6.6Hz), 1.20(3H,s), 1.25(3H,s), 3.35(1H, t, J=5.1Hz), 3.45(1H,brs), 4.01(1H, brs), 7.53-7.94(5H, m);
IR (neat) cm⁻¹: 3460(br), 2925, 1280, 1135, 1075;
MS(m/z): 439(M⁺+1), 60 (100%).

### Illustrative Examples 4: Synthesis of [3a(R)]-1(R)-[4(R),5-dihydroxy-1(S),5-dimethylhexyl]-4(S)-hydroxy-7a(R)-methyl-octahydro-1H-indene (compound 8a)

Sodium amalgam (5%, 5.01 g, 10.9 mmol) was added to a solution in THF (5.5 ml) and methanol (3.5 ml) of the [3a(R)]-1(R)-[4(R),5-dihydroxy-1(S),5-dimethyl-2-phenylsulfonylhexyl]-4(S)-hydroxy-7a(R)-methyl-octahydro-1H-indene (compound 7a;104 mg, 237 µmol) obtained in Example 2 followed by stirring for 15 hours at room temperature. Methanol (5.4 ml) and water (5.4 ml) were added to the reaction solution, followed by stirring for further 30 min. After removing the amalgam by decantation, the reaction solution was poured into a saturated aqueous solution of ammonium chloride, extracted twice with ethyl acetate and washed with brine. The organic layer was dried over magnesium sulfate and distilled under reduced pressure to remove the solvent; the resulting residue was purified by flash column chromatography (ethanol:dichloromethane = 0.3:5) to give the titled compound (68 mg, 96%) as colorless foam.
¹H NMR: δ 0.91(3H, d, J=6.3Hz), 0.94(3H, s), 1.21(3H, s), 1.24(3H, s), 3.33(1H, brt, J=5.4Hz), 4.08(1H, brs);
IR (neat) cm⁻¹: 3400(br), 2930;
MS(m/z): 298(M⁺), 135(100%).

### Illustrative Example 5: Synthesis of [3a(R)]-1(R)-[4(S),5-dihydroxy-1(S),5-dimethylhexyl]-4(S)-hydroxy-7a(R)-methyl-octahydro-1H-indene (compound 8b)

The same procedure as in Example 4 was repeated using the [3a(R)]-1(R)-[4(S),5-dihydroxy-1(S),5-dimethyl-2-phenylsulfonylhexyl]-4(S)-hydroxy-7a(R)-methyl-octahydro-1H-indene (compound 7b; 216 mg, 493 µmol) obtained in Example 3, THF (12 ml), methanol (7.5 ml) and sodium amalgam (5%, 12.4 g, 27.0 mmol). The titled compound was obtained as a colorless oil (100 mg, 68%).
¹H NMR: δ 0.92(3H, d, J=6.6Hz), 0.93(3H, s), 1.16(3H, s), 1.21(3H, s), 3.27(1H, dd, J=10.2, 2.0Hz), 4.07(1H, brd, J=2.6Hz);
IR (neat) cm⁻¹: 3400(br), 2930;
MS(m/z): 280 (M⁺- H₂O), 60(100%).

### Illustrative Example 6: Synthesis of [3a(R)]-4(S)-hydroxy-7a(R)-methyl-1(R)-[1(S)-methyl-3-[2,2,4,4-tetramethyl-1,3-dioxolane-2(R)-yl]propyl]-octahydro-1H-indene (compound 9a)

P-toluenesulfonic acid (TsOH) (6.5 mg, 34.2 µmol) was added to a solution in acetone (19.5 ml) of the [3a(R)]-1(R)-[4(R),5-dihydroxy-1(S),5-dimethylhexyl]-4(S)-hydroxy-7a(R)-methyl-octahydro-1H-indene (compound 8a; 86 mg, 289 µmol) obtained in Example 4 and 2,2-dimethoxypropane (DMP, 2.93 g, 28.1 mmol), followed by stirring for 15 hours at room temperature under argon atmosphere. The reaction mixture was mixed with sodium hydrogencarbonate (10 mg), concentrated under reduced pressure and extracted twice with dichloromethane. The organic layer was dried over magnesium sulfate and distilled under reduced pressure to remove the solvent; the resulting residue was purified by flash column chromatography (ethanol:dichloromethane = 0.2:5) to give a crude form of the titled compound (107 mg) as colorless prisms which were used in the following step without further purification.
¹H NMR: δ 0.93(3H, d, J=5.3Hz), 0.95(3H, s), 1.10(3H, s), 1.25(3H, s), 1.33(3H, s), 1.42(3H, s), 3.64(1H, dd, J=8.5, 3.7Hz), 4.08(1H, brd, J=2.4Hz);
IR (KBr) cm⁻¹: 3485(br), 2920;
MS(m/z): 338(M⁺), 323(100%).

### Illustrative Example 7: Synthesis of [3a(R)]-4(S)-hydroxy-7a(R)-methyl-1(R)-[1(S)-methyl-3-[2,2,4,4-tetramethyl-1,3-dioxolan-2(S)-yl]propyl]-octahydro-1H-indene (compound 9b)

The same procedure as in Example 6 was repeated using the [3a(R)]-1(R)-[4(S),5-dihydroxy-1(S),5-dimethylhexyl]-4(S)-hydroxy-7a(R)-methyl-octahydro-1H-indene (compound 8b; 100 mg, 289 µmol) obtained in Example 5, 2,2-dimethoxypropane (3.8 g, 36.5 mmol), acetone (23 ml), TsOH (15 mg, 78.9 µmol) and sodium hydrogencarbonate (20 mg). The titled compound was obtained as a colorless oil (76 mg, 67%).
¹H NMR: δ 0.93(3H, d, J=6.3Hz), 0.94(3H, s), 1.10(3H, s), 1.25(3H, s), 1.33(3H, s), 1.41(3H, s), 3.61(1H, dd, J=6.9, 5.9Hz), 4.08(1H, brs);
IR (neat) cm⁻¹: 3520(br), 2930;
MS(m/z): 323(M⁺-Me, 100%).

### Illustrative Example 8: Synthesis of [3a(R)]-4(S)-acetyloxy-1(R)-[1(S)-methyl-3-[2,2,4,4-tetramethyl-1,3-dioxolan-2(R)-yl]propyl]-7a(R)-methyl-octahydro-1H-indene (compound 10a)

Acetic anhydride (120 µl, 1.27 mmol) was added to a solution in dichloromethane (10 ml) of the crude [3a(R)]-4(S)-hydroxy-7a(R)-methyl-1(R)-[1(S)-methyl-3-[2,2,4,4-tetramethyl-1,3-dioxolan-2(R)-yl]propyl]-octahydro-1H-indene (compound 9a; 107 mg) obtained in Example 6, pyridine (128 µl) and DMAP (7 mg), followed by stirring for 4 hours at room temperature under argon atmosphere. The reaction mixture was poured into diluted hydrochloric acid, extracted with dichloromethane and washed with a saturated sodium carbonate solution. The organic layer was dried over magnesium sulfate and distilled under reduced pressure to remove the solvent; the resulting residue was purified by flash column chromatography (15% ethyl acetate/hexane) to give a crude form of the titled compound (91 mg, 83% yield from [3a(R)]-1(R)-[4(R),5-dihydroxy-1(S),5-dimethylhexyl]-4(S)-hydroxy-7a(R)-methyl-octahydro-1H-indene) as a colorless oil.
¹H NMR: δ 0.89(3H, s), 0.95(3H, d, J=6.3Hz), 1.10(3H, s), 1.25(3H, s), 1.33(3H, s), 1.42(3H, s), 2.04(3H, s), 3.64(1H, dd, J=8.8, 2.9Hz), 5.16 (1H, brs);
IR(neat)cm⁻¹:2940, 1735:
MS(m/z) :365 (M⁺-Me, 100%).

### Illustrative Example 9: Synthesis of [3a(R)]-4(S)-acetyloxy-1(R)-[1(S)-methyl-3-[2,2,4,4-tetramethyl-1,3-dioxolan-2(S)-yl]propyl]-7a(R)-methyl-octahydro-1H-indene (compound 10b)

The same procedure as in Example 8 was repeated using the [3a(R)]-4(S)-hydroxy-7a(R)-methyl-1(R)-[1(S)-methyl-3-[2,2,4,4-tetramethyl-1,3-dioxolan-2(S)-yl]propyl]-octahydro1H-indene (compound 9b; 76 mg, 225 µmol) obtained in Example 7, pyridine (500 µl), DMAP (3 mg), dichloromethane (3 ml) and acetic anhydride (250 µl, 2.64 mmol) to give the titled compound (82 mg, 96%) as a pale yellow oil.
¹H NMR: δ 0.89(3H, s), 0.94(3H, d, J=6.6Hz), 1.10(3H, s), 1.25(3H, s), 1.33(3H, s), 1.41(3H, s), 2.04(3H, s), 3.61 (1H, t, J=6.3Hz), 5.15 (1H, brs);
IR (neat) cm⁻¹: 2930, 1735;
MS(m/z): 365(M⁺-Me,100%).

### Illustrative Example 10: Synthesis of [3a(R)]-4(S)-acetyloxy-1(R)-[4(R),5-dihydroxy-1(S),5-dimethylhexyl]-7a(R)-methyl-octahydro-1H-indene (compound 11a)

One-percent iodine/methanol (2 ml) was added to the [3a(R)]-4(S)-acetyloxy-1(R)-[1(S)-methyl-3-[2,2,4,4-tetramethyl-1,3-dioxolan-2(R)-yl]propyl]-7a(R)-methyloctahydro-1H-indene (compound 10a; 46 mg, 121 µmol) obtained in Example 8, followed by heating under reflux for 4.5 hours. The reaction mixture was poured into a solution of 10% sodium thiosulfate (Na₂S₂O₃), concentrated under reduced pressure, extracted with ethyl acetate and washed twice with water. The organic layer was dried over magnesium sulfate and distilled under reduced pressure to remove the solvent; the resulting residue was purified by flash column chromatography (40% ethyl acetate/hexane) to give the titled compound (25 mg, 61%) as a colorless oil.
¹H NMR: δ 0.89(3H, s), 0.92(3H, d, J=6.6Hz), 1.16(3H, s), 1.21(3H, s), 2.04(3H, s), 3.32(1H, brt, J=5.9Hz), 5.14(1H, brs);
IR (neat) cm⁻¹: 3455(br), 2930, 1735;
MS(m/z): 280 (M⁺-Ac-H₂O), 59(100%).

### Illustrative Example 11: Synthesis of [3a(R)]-4(S)-acetyloxy-1(R)-[4(S),5-dihydroxy-1(S),5-dimethylhexyl]-7a(R)-methyl-octahydro-1H-indene (compound 11b)

A mixture of the [3a(R)]-4(S)-acetyloxy-1(R)-[1(S)-methyl-3-[2,2,4,4-tetramethyl-1,3-dioxolan-2(S)-yl]propyl]-7a(R)-methyl-octahydro-1H-indene (compound 10b; 34 mg, 89.5 µmol) obtained in Example 9, acetic acid (240 µl), water (1.99 ml) and ethanol (2.6 ml) was stirred at 70°C for 5 days. The reaction mixture was poured into a saturated sodium hydrogencarbonate solution, extracted twice with ethyl acetate and washed with brine. The organic layer was dried over magnesium sulfate and distilled under reduced pressure to remove the solvent; the resulting residue was purified by flash column chromatography (40% ethyl acetate/hexane) to give the titled compound (25 mg, 83%) as a colorless oil.
¹H NMR: δ 0.88(3H, s), 0.93(3H, d, J=6.6Hz), 1.16(3H, s), 1.22(3H, s), 2.04(3H, s), 3.23-3.32(1H, m), 5.15(1H, brs);
IR (neat) cm⁻¹: 3460(br), 2935, 1730:
MS(m/z): 340(M⁺), 135(100%).

### Illustrative Example 12: Synthesis of [3a(R)]-4(S)-acetyloxy-1(R)-[4(R),5-bis(t-butyldimethylsilyloxy)-1(S),5-dimethylhexyl]-7a(R)-methyl-octahydro-1H-indene (compound 12a)

Under cooling with ice, t-butyldimethylsilyltrifluoromethanesulfonate (TBSOTf) (466 µl, 2.03 mmol) was added to a solution in dichloromethane (10 ml) of the [3a(R)]-4(S)-acetyloxy-1(R)-[4(R),5-dihydroxy-1(S),5-dimethylhexyl]-7a(R)-methyl-octahydro-1H-indene (compound 11a; 46 mg, 135 µmol) obtained in Example 10, and 2,6-lutidine (355 µl, 3.03 mmol), followed by stirring at room temperature for 1.5 hours. The reaction mixture was poured into ice-cooled 1N hydrochloric acid, extracted with dichloromethane and washed with saturated sodium carbonate solution. The organic layer was dried over magnesium sulfate and distilled under reduced pressure to remove the solvent; the resulting residue was purified by flash column chromatography (6% ethyl acetate/hexane) to give the titled compound (71 mg, 92%) as a colorless oil.
¹H NMR: δ 0.04(3H, s), 0.06(3H, s), 0.07(3H, s), 0.08(3H, s), 0.86(9H, s), 0.89(9H, s), 1.11(3H, s), 1.19(3H, s), 2.04(3H, s), 3.22(1H, brs), 5.15(1H, brs);
IR (neat) cm⁻¹: 2945, 1745:
MS(m/z): 553(M⁺-Me), 173(100%).

### Illustrative Example 13: Synthesis of [3a(R)]-4(S)-acetyloxy-1(R)-[4(S),5-bis(t-butyldimethylsilyloxy)-1(S),5-dimethylhexyl]-7a(R)-methyl-octahydro-1H-indene (compound 12b)

The same procedure as in Example 12 was repeated using the [3a(R)]-4(S)-acetyloxy-1(R)-[4(S),5-dihydroxy-1(S),5-dimethylhexyl]-7a(R)-methyl-octahydro-1H-indene (compound 11b; 31 mg, 91.2 µmol) obtained in Example 11, 2,6-lutidine (64 µl, 550 µmol), dichloromethane (2 ml) and TBSOTf (84 µl, 366 mmol) to give the titled compound (47 mg, 91%) as a colorless oil.
¹H NMR : δ 0.04(3H, s), 0.06(3H, s), 0.07(3H, s), 0.08(3H, s), 0.85(9H, s), 0.88(9H, s), 1.10(3H, s), 1.19(3H, s), 2.04(3H, s), 3.18(1H, dd, J=7.4, 2.3Hz), 5.15(1H, brs):
IR (neat) cm⁻¹: 2945, 1735:
MS(m/z): 553(M⁺-Me), 173(100%)

### Illustrative Example 14: Synthesis of [3a(R)]-1(R)-[4(R),5-bis(t-butyldimethylsilyloxy)-1(S),5-dimethylhexyl]-4(S)-hydroxy-7a(R)-methyl-octahydro-1H-indene (compound 3a)

The [3a(R)]-4(S)-acetyloxy-1(R)-[4(R),5-bis(t-butyldimethylsilyloxy)-1(S),5-dimethylhexyl]-7a(R)-methyl-octahydro-1H-indene (compound 12a; 71 mg, 125 µmol) obtained in Example 12 was dissolved in THF (2 ml). The resulting solution was added dropwise to a suspension of lithium aluminum hydride (LiAlH₄) (20 mg, 528 µmol) in THF (1 ml) under cooling with ice, followed by stirring for 1.5 hours at the same temperature under argon atmosphere. Five droplets of 1N sodium hydroxide (NaOH) were added to the reaction mixture and then an aqueous Rochelle salt solution was added. The resulting mixture was extracted twice with ethyl acetate and washed with brine. The organic layer was dried over magnesium sulfate and distilled under reduced pressure to remove the solvent; the resulting residue was purified by flash column chromatography (12% ethyl acetate/hexane) to give the titled compound (63 mg, 96%) as a colorless oil.
¹H NMR: δ 0.03(3H, s), 0.06(3H, s), 0.07(3H, s), 0.08(3H, s), 0.85(9H, s), 0.89(9H, s), 1.11(3H, s), 1.18(3H, s), 3.23(1H, brs), 4.08(1H, brs):
IR (neat) cm⁻¹: 3425(br), 2925;
MS(m/z): 511(M⁺-Me), 173 (100%).

### Illustrative Example 15: Synthesis of [3a(R)]-1(R)-[4(S),5-bis(t-butyldimethylsilyloxy)-1(S),5-dimethylhexyl]-4(S)-hydroxy-7a(R)-methyl-octahydro-1H-indene (compound 3b)

The same procedure as in Example 14 was repeated using LiA1H₄ (6.3 mg, 166 µmol), THF (3 ml) and the [3a(R)]-4(S)-acetyloxy-1-(R)-[4(S),5-bis(t-butyldimethylsilyloxy)-1(S),5-dimethylhexyl]-7a(R)-methyl-octahydro-1H-indene (compound 12b; 47 mg, 82.7 µmol) obtained in Example 13. The titled compound (44 mg, 100%) was obtained as a colorless oil.
¹H NMR: δ 0.04(3H, s), 0.067(3H, s), 0.072(3H, s), 0.08(3H, s), 0.85(9H, s), 0.89(9H, s), 0.92(3H, d, J=6.3Hz), 1.11(3H, s), 1.19(3H, s), 3.18(1H, dd, J=7.6, 2.3Hz), 4.00(1H, brs):
IR (neat) cm⁻¹:3430(br), 2920;
MS(m/z): 511(M⁺-Me), 173(100%).

The reactions performed in the above Examples 1 to 15 are shown below:

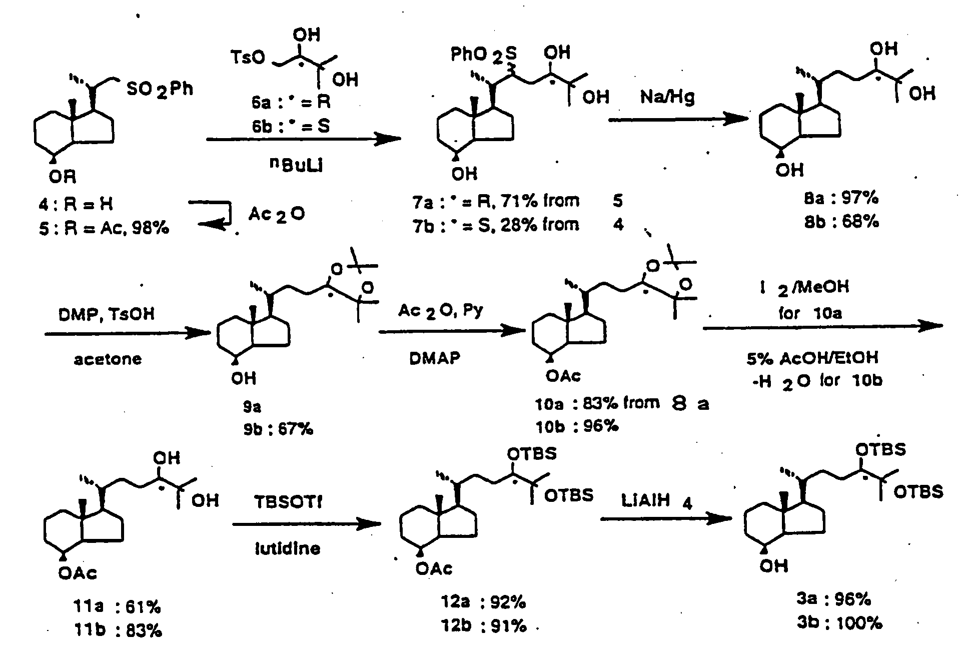

### Illustrative Example 16: Synthesis of [3a(R)]-1(R)-[4(R),5-bis(t-butyldimethylsilyloxy)-1(S),5-dimethylhexyl]-7a(R)-methyloctahydro-1H-inden-4-one (compound 2a)

Tetra-n-propylammonium perruthenate (TPAP, 0.6 mg, 1.71 µmol) was added to a suspension in dichloromethane (1.3 ml) of the [3a(R)]-1(R)-[4(R),5-bis(t-butyldimethylsilyloxy)-1(S),5-dimethylhexyl]-4(S)-hydroxy-7a(R)-methyl-octahydro-1H-indene (compound 3a; 16.4 mg, 31.2 µmol) obtained in Example 14, 4-methyl-morpholine N-oxide (NMO) (5.5 mg, 46.9 µmol) and molecular sieve 4A (6 mg), followed by stirring for 1 hour at room temperature under argon atmosphere. The reaction mixture was poured into a 10% Na₂S₂O₃ solution, extracted twice with dichloromethane and washed with brine. The organic layer was dried over magnesium sulfate and distilled under reduced pressure to remove the solvent; the resulting residue was purified by flash column chromatography (7% ethyl acetate/hexane) to give the titled compound (14 mg, 86%) as a colorless oil.
¹H NMR: δ 0.04(3H, s), 0.06(3H, s), 0.07(3H, s), 0.08(3H, s), 0.63(3H, s), 0.85(9H, s), 0.89(9H, s), 0.95(3H, d, J=5.9Hz), 1.11(3H, s), 1.19(3H, s), 2.45(1H, dd, J=11.5, 7.6Hz), 3.23 (1H, brs);
IR(neat)cm⁻¹: 2950, 1715;
MS(m/z): 524(M⁺), 173(100%).

### Reference Example 1: Synthesis of 1(S),3(R),24(R),25-tetrakis(t-butyldimethylsilyloxy)-9,10-secocholesta 5,7,10(19)-triene (compound 15a)

Under argon atmosphere at -80°C, n-butyllithium (1.47 mol/1,88 µl, 129 µmol) was added dropwise to a solution in THF (0.5 ml) of 2-[3',5'-bis(t-butyldimethylsilyloxy)-2'-methylenecyclohexylidene]-1-diphenylphosphorylethane (compound 13, S.Hatakeyama et al., The Journal of Organic Chemistry, 54, 3515 (1989)) (45 mg, 77.3 µmol), followed by stirring at the same temperature for 5 min. A solution in THF (300 µl) of the [3a(R)]-1(R)-[4(R),5-bis(t-butyldimethylsilyloxy)-1(S),5-dimethylhexyl]-7a(R)-methyloctahydro-1H-inden-4-one (compound 2a; 8.7 mg, 16.6 µmol) obtained in Example 16 was added dropwise to the resulting mixture, followed by stirring at the same temperature for 1 hour and then at room temperature for 10 min. The reaction mixture was poured into brine and extracted twice with ethyl acetate. The organic layer was dried over magnesium sulfate and distilled under reduced pressure to remove the solvent; the resulting residue was purified by preparative thin-layer chromatography (TLC) (3% ethyl acetate/hexane) to give the titled compound (7 mg, 48%) as a colorless oil.
¹H NMR: δ 0.04(3H, s), 0.06(3H, s), 0.07(15H, s), 0.08(3H, s), 0.53(3H, s), 0.86(9H, s), 0.877(9H, s), 0.881(9H, s), 0.89(9H, s), 1.11(3H, s), 1.19(3H, s), 3.23(1H, brs), 4.15-4.23(1H, m), 4.36-4.41(1H, m), 4.87(1H, brd, J=2.6Hz), 5.00(1H, s), 6.02(1H, d, J=11.1Hz), 6.24(1H, d, J=11.1Hz);
IR (neat) cm⁻¹: 2940; MS(m/z): 888(M⁺), 173(100%);
UV λ max nm: 264, min nm: 227.

### Reference Example 2: Synthesis of 1(S),3(R),24(R),25-tetrahydroxy-9,10-secocholesta-5,7,10(19)-triene (compound 14a)

The 1(S),3(R),24(R),25-tetrakis(t-butyldimethylsilyloxy)-9,10-secocholesta- 5,7,10(19)-triene (compound 15a; 7 mg, 7.88 µmol) obtained in Reference Example 1 was dissolved in 1,3-dimethyl-2-imidazolidinone (DMI) (3 ml). To the resulting solution, tetra-n-butylammonium fluoride (TBAF) (1 mol/l, 300 µl, 300 µmol) was added, followed by stirring for 2 hours under argon atmosphere at 105°C. The reaction mixture was poured into water, extracted twice with ethyl acetate and washed twice with water. The organic layer was dried over magnesium sulfate and distilled under reduced pressure to remove the solvent; the resulting residue was purified by preparative thin-layer chromatography (TLC) (ethanol:hexane = 3:25) to give the titled compound (2.7 mg, 79%) as a white powder.
¹H NMR: δ 0.55(3H, s), 0.94(3H, d, J=5,9Hz), 1.17(3H, s), 1.22(3H, s), 2.31(1H, dd, J=13.2, 6.6Hz), 2.60(1H, dd, J=13.5, 3.6Hz), 2.82(1H, dd, J=10.6, 3.6Hz), 3.31-3.37(1H, m), 4.18-4.27(1H, m), 4.40-4.48(1H, m), 5.00(1H, s), 5.33(1H, s), 6.02(1H, d, J=11.4Hz), 6.38(1H, d, J=11.4Hz);
IR (KBr) cm⁻¹: 3380(br), 2925;
MS(m/z): 432(M⁺), 134(100%);
UV λ max nm:264,min nm:227.

The reactions involved in the above illustrative Example 16 and Reference Examples 1 and 2 are shown below:

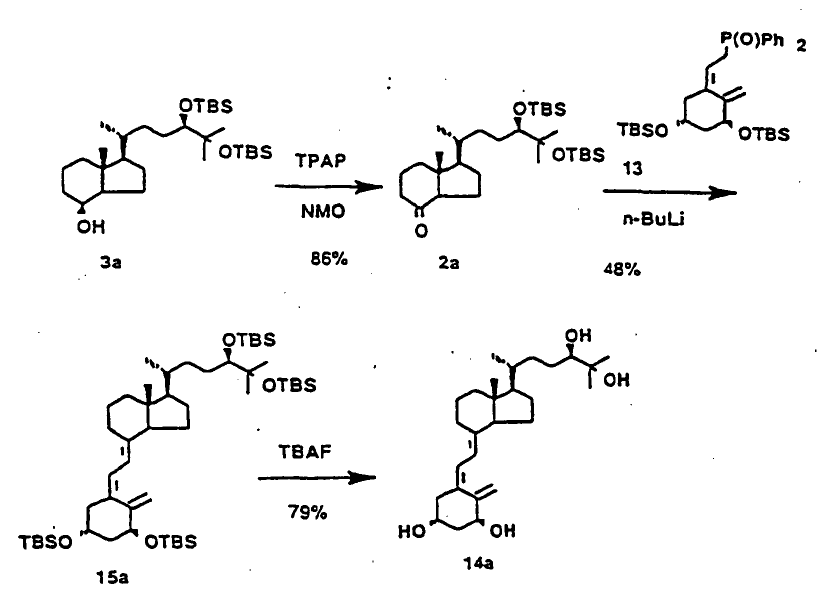

### Illustrative Example 17: Synthesis of [3a(R)]-1(R)-[4(S),5-bis(t-butyldimethylsilyloxy)-1(S),5-dimethylhexyl]-7a(R)-methyloctahydro-1H-inden-4-one

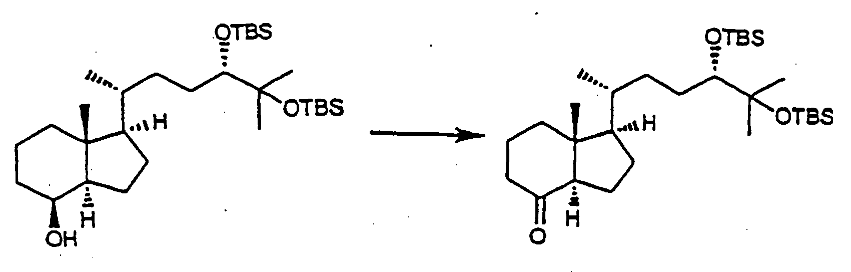

NMO (7.8 mg, 0.066 mmol) and 4A molecular sieve (19.0 mg) were added to a solution of [3a(R)]-1(R)-[4(S),5-bis(t-butyldimethylsilyloxy)-1(S),5-dimethylhexyl]-4(S)-hydroxy-7a(R)-methyl-octahydro-1H-indene (23.7 mg, 0.044 mmol) in dichloromethane (3 ml), followed by stirring for 1 hour at room temperature under argon atmosphere; TPAP (0.8 mg, 0.0023 mmol) was further added. After 5 hours, the reaction solution was filtered with Celite and the filtrate was concentrated under reduced pressure. The resulting residue was purified by column chromatography (n-hexane:ethyl acetate = 5:1) to give the titled compound (23.7 mg, 103%) as a colorless oil.
[α]_{D}²⁴-5.3° (c 1.185, CHCl₃);
IR(neat) 1716, 1467, 1252 cm⁻¹;
¹H NMR (300 MHz, CDCl₃) δ 3.18(dd, 1H, J=2.4Hz, 7.5Hz), 2.43(dd, 1H, J=7.5Hz, 11.1Hz), 1.20-2.28(m, 16H), 1.19(s, 3H), 1.10(s, 3H), 0.94(d, 3H, J=6.3Hz), 0.88(s, 9H), 085(s, 9H), 0.63(s, 3H), 0.08(s, 3H), 0.07(s, 3H), 0.06(s, 3H), 0.04(s, 3H);
¹³C NMR (75 MHz, CDCl₃) δ 212.2, 81.2, 76.4, 62.1, 56.8, 50.0, 41.1, 39.1, 36.2, 34.1, 29.7, 29.0, 27.8, 26.2, 26.0, 24.2 23.2, 19.2, 18.8, 18.3, 18.2, 12.5, -1.8, -1.9, -3.1, -3.7. MS(EI)m/z 173, 509(M⁺-CH₃), 467(M⁺-57),
HRMS (EI) Calcd. for C₂₉H₅₇O₃Si₂ 509.3846; Found: 509.3852.

### Illustrative Example 18: Synthesis of [3a(R)]-1(R)-[4(R),5-bis(t-butyldimethylsilyloxy)-1(S),5-dimethylhexyl]-7a(R)-methyloctahydro-1H-inden-4-one

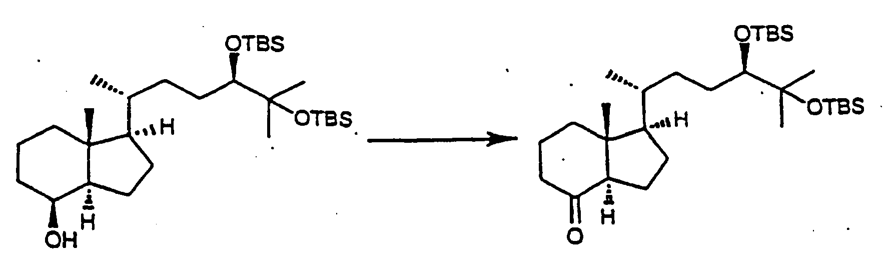

NMO (7.0 mg, 0.059 mmol) and 4A molecular sieve (14.0 mg) were added to a solution of [3a(R)]-1(R)-[4(R),5-bis(t-butyldimethylsilyloxy)-1(S),5-dimethylhexyl]-4(S)-hydroxy-7a(R)-methyl-octahydro-1H-indene (15.4 mg, 0.029 mmol) in dichloromethane (2 ml), followed by stirring for 1 hour at room temperature under argon atmosphere; TPAP (0.8 mg, 0.0023 mmol) was further added. After 2.5 hours, the reaction solution was filtered with Celite and the filtrate was concentrated under reduced pressure. The resulting residue was purified by column chromatography (n-hexane:ethyl acetate = 5:1) to give the titled compound (14.8 mg, 96%) as a colorless oil.
[α]_{D}²³+ 17.0° (c 0.83, CHCl₃) ;
IR (neat) 1716, 1460, 1252 cm⁻¹;
¹H NMR (300MHz, CDCl₃) δ 3.18-3.24 (m, 1H), 2.45(dd, 1H, J=7.5Hz, 11.1Hz), 1.20-2.34(m, 16H), 1.18(s, 3H), 1.11(s, 3H), 0.94(d, 3H, J=5.7Hz), 0.89(s, 9H), 0.85(s, 9H), 0.63(s, 3H), 0.08(s, 3H), 0.07(s, 3H), 0.06(s, 3H), 0.04(s, 3H);
¹³C NMR (75MHz, CDCl₃) δ 212.2, 81.0, 76.4, 62.1, 56.7, 50.0, 41.1, 39.1, 36.3, 33.8, 29.6, 29.0, 27.6, 26.2, 25.9, 24.2, 23.6, 19.2, 18.9, 18.3, 18.2. 12.6, -1.8, -1.9, -3.1, -3.9. MS (EI) m/z 173, 509 (M⁺-CH₃), 467 (M⁺-57),
HRMS(EI) Calcd. for C₂₉H₅₇O₃Si₂ 509.3846; Found 509.3845.

### Illustrative Example 19: Synthesis of [3a(R)]-1(R)-[4(S),5-bis(t-butyldimethylsilyloxy)-1(S),5-dimethylhexyl]-4(E)-bromomethylene-7a(R)-methyl-octahydro-1H-indene

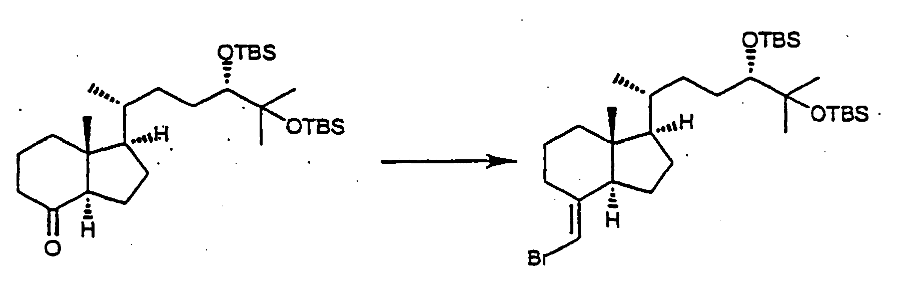

To a solution of (bromomethylene)triphenylphosphonium bromide (159.3 mg, 0.37 mmol) in THF (0.9 ml), sodium bis(trimethylsilyl)amide (in THF at 1M, 355 µl, 0.36 mmol) was added at -60°C, followed by stirring for 1 hour under argon atmosphere. Immediately after adding a solution in THF (0.3 ml) of the [3a(R)]-1(R)-[4(S),5-bis(t-butyldimethylsilyloxy)-1(S),5-dimethylhexyl]-7a(R)-methyloctahydro-1H-inden-4-one (23.9 mg, 0.046 mmol) obtained in Example 17, the reaction temperature was returned to room temperature. After 1 hour, the resulting mixture was diluted with n-hexane and filtered with silica gel; the filtrate was concentrated under reduced pressure. The resulting residue was purified by preparative TLC (n-hexane) to give the titled compound (15.6 mg, 57%) as a yellow oil.
[α]_{D}²³ +47.31° (c 0.78, CHCl₃);
IR (neat) 1466, 1252 cm⁻¹;
¹H NMR (300 MHz, CDCl₃) δ 5.64(s, 1H), 3.18(dd, 1H, J=2.1Hz, 7.5Hz), 2.82-2.91(m, 1H), 1.00-2.16(m, 16H), 1.19(s, 3H), 1.10(s, 3H), 0.92(d, 3H, J=6.0Hz), 0.88(s, 9H), 0.85(s, 9H), 0.56(s, 3H), 0.08(s, 3H), 0.07(s, 3H), 0.06(s, 3H), 0.04(s, 3H);
¹³C NMR (75 MHz, CDCl₃) δ 145.3, 97.4, 81.2, 76.4, 56.0, 55.9, 45.6, 40.0, 36.7, 34.2, 31.2, 29.7, 29.0, 27.9, 26.2, 25.9, 23.2, 22.7, 22.1, 18.9, 18.3, 18.2, 11.9, -1.8, -1.9, -3.1, -3.7.
MS(EI)m/z 73, 585 (M⁺-CH₃), 543 (M⁺-57),
HRMS(EI) Calcd. for C₂₇H₅₂O₂Si₂Br 543.2689; Found 543.2692.

### Illustrative Example 20: Synthesis of [3a(R)]-1(R)-[4(R),5-bis(t-butyldimethylsilyloxy)-1(S),5-dimethylhexyl]-4(E)-bromomethylene-7a(R)-methyl-octahydro-1H-indene

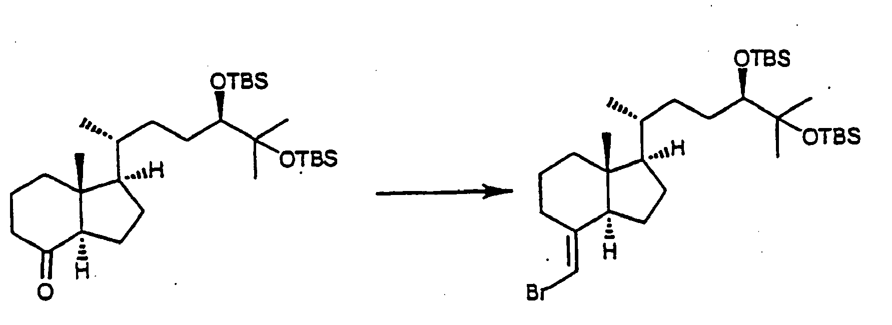

To a solution of (bromomethylene)triphenylphosphonium bromide (130.5 mg, 0.30 mmol) in THF (1.0 ml), sodium bis(trimethylsilyl)amide (in THF at 1M, 290 µl, 0.29 mmol) was added at -60°C, followed by stirring for 1 hour under argon atmosphere. Immediately after adding a solution in THF (0.3 ml) of the [3a(R)]-1(R)-[4(R),5-bis(t-butyldimethylsilyloxy)-1(S),5-dimethylhexyl]-7a(R)-methyloctahydro-1H-inden-4-one (19.6 mg, 0.037 mmol) obtained in Example 18 the reaction temperature was returned to room temperature. After 1 hour, the resulting mixture was diluted with n-hexane and filtered with silica gel; the filtrate was concentrated under reduced pressure. The resulting residue was purified by preparative TLC (n-hexane) to give the titled compound (11.0 mg, 49%) as a yellow oil.
[α]_{D}²¹ -106.18° (c 0.55, CHCl₃);
IR (neat) 1467, 1253cm⁻¹;
¹H NMR (300MHz, CDCl₃) δ 5.64(s, 1H), 3.18-3.24(m, 1H), 2.82-2.91(m, 1H), 1.20-2.04(m, 16H), 1.18(s, 3H), 1.11(s, 3H), 0.92(d, 3H, J=6.0Hz), 0.89(s, 9H), 0.85(s, 9H), 0.56(s, 3H), 0.08(s, 3H), 0.07(s, 3H), 0.06(s, 3H), 0.04(s, 3H);
¹³C NMR (75MHz, CDCl₃) δ 145.3, 97.4, 81.1, 76.4, 56.0, 55.9, 45.6, 40.0, 36.8, 33.9, 31.2, 29.6, 29.1, 27.7, 26.2, 26.0, 23.6, 22.7, 22.2, 19.0, 18.3, 18.2, -1.8, -1.9, -3.1, -3.9.
MS (EI) m/z 73, 585 (M⁺-CH₃), 543 (M⁺-57),
HRMS(EI) Calcd. for C₃₀H₅₈O₂Si₂Br 585.3150; Found for C₃₀H₅₈O₂Si₂Br 585.3134.

### Example 21: Synthesis of 3,5-bis(t-butyldimethylsilyloxy)-4-(3-t-butyldimethylsilyloxypropoxy)-1-octen-7-ine

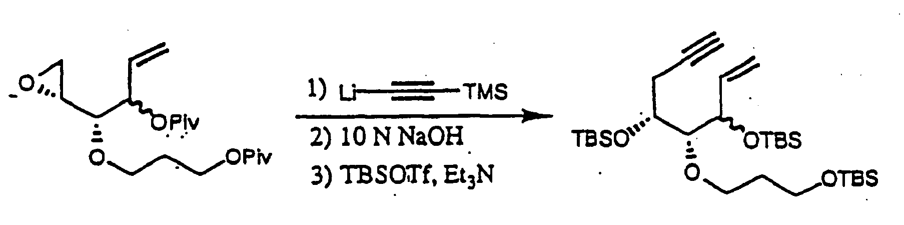

A 1.59M solution of n-butyllithium in n-hexane (35.2 ml, 56.0 mmol) was added to a solution of trimethylsilylacetylene (8.4 ml, 11.9 mmol) in THF (30 ml) at -78°C, followed by stirring for 30 min.; boron trifluoride diethyl ether (11.9 mmol) was further added and the mixture was stirred for 1 hour. To the resulting mixture, a solution of a 1:1 mixture of (4R)- and (4S)- epimers of 1,2-epoxy-4-pivaloyloxy-3-(3'-pivaloyloxypropoxy)-5-hexene (4.24 g, 11.9 mmol) in THF (65 ml) was added dropwise at -78°C, followed by stirring for 2 hours. A saturated aqueous solution of NaHCO₃ was added to the resulting reaction solution, extracted with ethyl acetate, washed with brine, dried (MgSO₄) and then distilled under reduced pressure to remove the solvent, giving a yellow oil (5.1 g).

Methanol (80 ml) and 10 N aqueous sodium hydroxide (35 ml) were added to the above yellow oil (5.1 g), followed by stirring for one hour and a half at room temperature. The resulting mixture was neutralized with concentrated hydrochloric acid under cooling with ice, azeotropically distilled with toluene to remove water and extracted with THF. The extract was dried (MgSO₄) and then distilled under reduced pressure to remove the solvent; the resulting residue was purified on a short column (ethyl acetate) to give a triol compound, 3,5-dihydroxy-4-(3-hydroxypropoxy)-1-octen-7-ine (1.73 g).

The above triol compound (1.73 g) was dissolved in dichloromethane (100 ml) and mixed with triethylamine (11.3 ml, 0.4 mol) and TBSOTf (10.7 ml, 40.4 mmol) at 0°C. The mixture was stirred for one hour and a half at 0°C, mixed with a saturated aqueous NaHCO₃ solution and extracted with dichloromethane. The extract was washed with brine, dried (MgSO₄) and distilled under reduced pressure to remove the solvent. The resulting residue was purified by flash column chromatography (n-hexane: ethyl acetate = 50:1) to give Fraction 1 (epimer mixture = 1.1265 g), Fraction 2 (epimer mixture = 2.4804 g) and Fraction 3 (S form = 334.8 mg) (yeild of the epimer mixtures = 3.9417 g, 60% from the epoxied). Fraction 1 was further separated by flash column chromatography (n-hexane: ethyl acetate = 50:1) to give an R form (169.3 mg) and an epimer mixture (789.4 mg).
R form
[α]_{D}²⁵ +15.4° (c 0.52, CHCl₃);
IR (neat) 3314, 1468, 1254cm⁻¹;
¹H NMR (300 MHz, CDCl₃) δ 5.89(ddd, 1H, J=6.0Hz, 10.5Hz, 17.4Hz), 5.26(dt, 1H, J=1.5Hz, 17.1Hz), 5.14(dt, 1H, J=1.5Hz, 10.5Hz), 4.18(t, 1H, J=6.3Hz), 3.98(ddd, 1H, J=1.8Hz, 4.8Hz, 7.2Hz), 3.60-3.82(m, 4H), 3.27(dd, 1H, J=2.1Hz, 6.6Hz), 2.46(ddd, 1H, J=3.0Hz, 5.1Hz, 17.1Hz), 1.92(t, 1H, 2.7Hz), 1.78(quint, 2H, J=6.0Hz), 0.89-0.90(m, 2H), 0.10(s, 3H), 0.08(s, 3H), 0.06(s, 3H), 0.04(s, 6H), 0.03(s, 3H);
¹³C NMR(75MHz, CDCl₃) δ 138.5, 116.0, 86.9, 83.2, 74.4, 72.1, 69.5, 60.5, 33.7, 26.1, 26.0, 23.3, 18.5, 18.4, -4.3, -4.4, -4.5, -5.2.
MS(EI)m/z 147(100%), 556, 541, 499.
HRMS (EI) Calcd. for C₂₈H₅₇O₄Si₃ 541.3565; Found for C₂₈H₅₇O₄Si₃ 541.3547.
S form
[α]_{D}²⁵ -2.3° (c0.80, CHCl₃);
IR(neat) 3314, 1468, 1254 cm⁻¹;
¹H NMR (300 MHz, CDCl₃) δ 5.88(ddd, 1H, J=6.0Hz, 10.2Hz, 17.4Hz), 5.26(dt, 1H, J=1.5Hz, 17.1Hz), 5.14(dt, 1H, J=1.5Hz, 10.5Hz), 4.27(dd, 1H, J=4.5Hz, 6.0Hz), 3.85(dt, 1H, J=4.2Hz, 5.7Hz), 3.74(ddd, 2H, J=3.0Hz, 6.3Hz, 12.9Hz), 3.67(t, 2H, J=6.3Hz), 3.36(dd, 1H, J=4.5Hz, 5.7Hz), 2.53(ddd, 1H, J=2.7Hz, 5.7Hz, 17.4Hz), 1.93(t, 1H, J=2.7Hz), 1.74(quint, 2H, J=6.3Hz), 0.91(s, 9H), 0.90(s, 9H), 0.88(s, 9H), 0.03-0.12(m, 18H);
¹³C NMR (75MHz, CDCl₃) δ 138.2, 116.1, 85.7. 82.3, 74.4, 71.0, 69.8, 69.7, 60.3, 33.7, 26.1, 26.0, 23.3, 18.5, 18.2, 18.2, -4.2, -4.3, -4.4, -4.7, -5.2.

### Illustrative Example 22: Synthesis of (5Z,7E)-(1R,2R,3R,24S)-1,3,24,25-tetrakis(t-butyldimethylsilyloxy)-2-(3-t-butyldimethylsilyloxypropoxy)-9,10-secocholesta-5,7,10(19)-triene

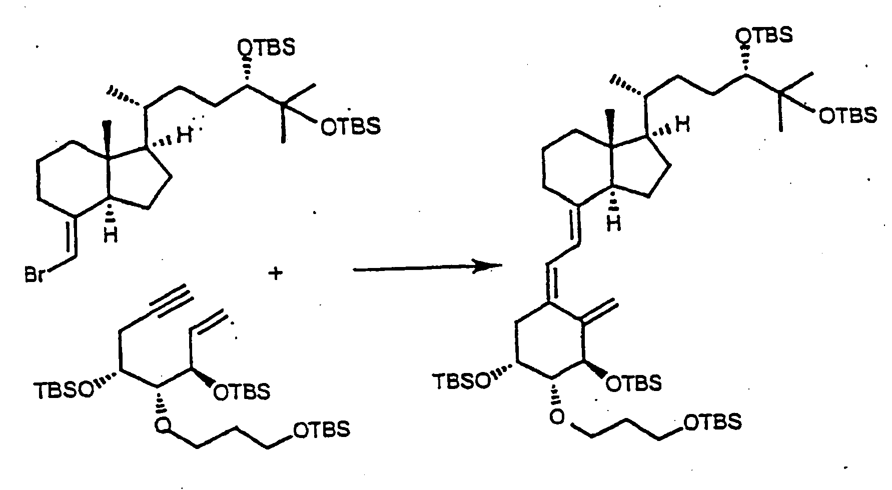

A mixture solution of triphenylphosphine (2.4 mg, 0.0092 mmol) and tris(dibenzylideneacetone)(chloroform)dipalladium(0) (1.7 mg, 0.0016 mmol) in toluene (0.2 ml) and triethylamine (0.3 ml) was stirred at room temperature for 10 min. and then mixed with a mixture solution in toluene (0.2 ml) of the [3a(R)]-1(R)-[4(S),5-bis(t-butyldimethylsilyloxy)-1(S),5-dimethylhexyl]-4(E)-bromomethylene-7a(R)-methyl-octahydro-1H-indene (15.6 mg, 0.026 mmol) obtained in Example 19 and the 3(R)-3,5-bis(t-butyldimethylsilyloxy)-4-(3-t-butyldimethylsilyloxypropoxy)-1-octen-7-ine (9.6 mg, 0.017 mmol) obtained in Example 21, followed by heating under reflux for 4 hours. The resultant was diluted with n-hexane, filtered with silica gel and concentrated under reduced pressure. The resulting residue was purified by preparative TLC (n-hexane:benzene = 2:1) to give the titled compound (6.2 mg, 34%) as a colorless oil.
[α]_{D}²³ + 10.7° (c0.31, CHCl₃);
IR (neat) 1467, 1252 cm⁻¹;
¹H NMR (300MHz, CDCl₃) δ 6.22(d, 1H, J=10.5Hz), 6.00(d, 1H, J=10.8Hz), 5.26(br s, 1H), 4.98(br s, 1H), 4.16-4.26(m, 2H), 3.58-3.76(m, 4H), 3.16-3.24(m, 2H), 2.81(br d, 1H, J=13.5Hz), 2.46(br dd, 1H, J=11.6Hz, 8.1Hz), 2.22(br dd, 1H, J=12.6Hz, 3.8Hz), 1.20-2.05(m, 18H), 1.19(s, 3H), 1.11(s, 3H), 0.82-0.94(m, 48H), 0.52(s, 3H), 0.02-0.10(m, 30H).

### Illustrative Example 23: Synthesis of (5Z,7E)-(1R,2R,3R,24R)-1,3,24,25-tetrakls(t-butyldimethylsilyloxy)-2-(3-t-butyldimethylsilyloxypropoxy)-9,10-secocholesta-5,7,10(19)-triene

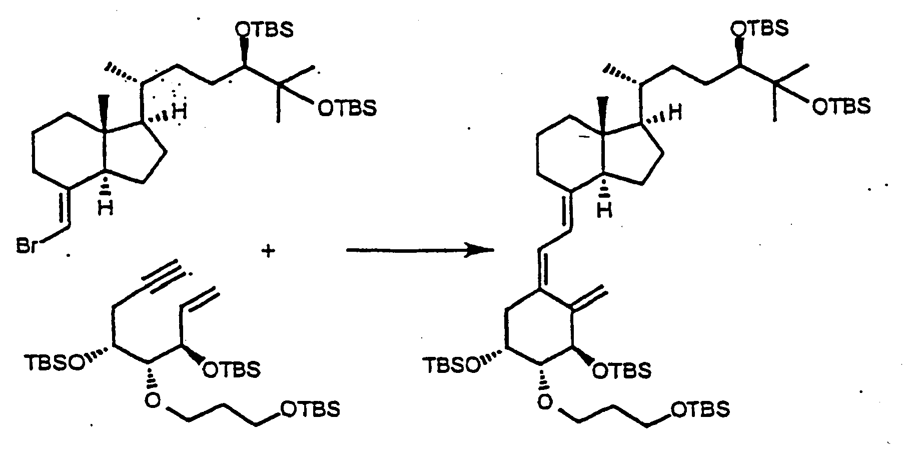

The same procedure as in Example 22 was repeated by replacing the [3a(R)]-1(R)-[4(S),5-bis(t-butyldimethylsilyloxy)-1(S),5-dimethylhexyl]-4(E)-bromomethylene-7a(R)-methyl-octahydro-1H-indene with the [3a(R)]-1(R)-[4(R),5-bis(t-butyldimethylsilyloxy)-1(S),5-dimethylhexyl]-4(E)-bromomethylene-7a(R)-methyl-octahydro-1H-indene obtained in Example 20. The titled compound (6.3 mg, 36%) was obtained as a colorless oil.
[α]_{D}²³ + 25.8° (c 0.28, CHCl₃);
IR (neat) 1467, 1252 cm⁻¹;
¹H NMR (300 MHz, CDCl₃) δ 6.22(d, 1H, J=10.5Hz), 6.00(d, 1H, J=10.8Hz), 5.26(br s, 1H), 4.98(br s, 1H), 4.16-4.26(m, 2H), 3.58-3.76(m, 4H), 3.22(m, 2H), 2.81(br d, 1H, J=12.3Hz), 2.46(br dd, 1H, J=12.9Hz, 7.8Hz), 2.23(br dd, 1H, J=12.6Hz, 3.3Hz), 1.20-2.05(m, 18H), 1.18(s, 3H), 1.11(s, 3H), 0.82-0.94(m, 48H), 0.52(s, 3H), 0.02-0.10(m, 30H).

### Illustrative Example 24: Synthesis of (5Z, 7E)-(1R, 2R, 3R, 24S)-2-(3-hydroxypropoxy)-9,10-secocholesta-5,7,10(19)-triene-1,3,24,25-tetraol

Under argon atmosphere, a 1M-TBAF-THF solution (237 µl, 237 µmol) was added to a solution of (5Z, 7E)-(1R, 2R, 3R, 24S)-1, 3, 24, 25-tetrakis(t-butyldimethylsilyloxy)-2-(3-t-butyldimethylsilyloxypropoxy)-9,10-secocholesta-5,7,10(19)-triene (compound 20a; 5.1 mg, 4.73 µmol) in toluene (0.5 ml), followed by stirring for 2 hours under heating at an external temperature of 105°C. After cooling to room temperature, the reaction solution was diluted with ethyl acetate, washed twice with water and once with brine, dried over anhydrous magnesium sulfate and distilled under reduced pressure to remove the solvent. The resulting residue was purified by preparative TLC (the thickness of silica gel: 0.25 mm, dichloromethane;ethanol = 5:1) to give the titled compound (compound 1a; 1.45 mg, 60%) as a colorless oil.
[α]²⁴_{D} -49° (c0.07, EtOH);
¹H NMR(270 MHz, CDCl₃) δ 0.55(s, 3H), 0.95(d, J=6.3Hz, 3H), 1.16(s, 3H), 1.22(s, 3H), 2.42(brd, J=13.9Hz, 1H), 2.55(dd, J=14.5, 4.0Hz, 1H), 2.75-2.87(m, 1H), 3.20-3.37(m, 2H), 3.68-3.78(m, 1H), 3.80-3.99(m, 3H), 4.20-4.36(m, 2H), 5.08(brs, 1H), 5.50(brs, 1H), 6.04(d, J=11.2Hz, 1H), 6.36(d, J=11.2Hz, 1H);
IR(neat,cm⁻¹) 3400, 2947, 2929, 2873, 1378, 1107, 1072;
UV(EtOH) λ max 264 nm.

### Illustrative Example 25: Synthesis of (5Z, 7E)-(1R, 2R, 3R, 24R)-2-(3-hydroxypropoxy)-9,10-secocholesta-5,7,10(19)-triene-1,3,24,25-tetraol

The same procedure as in Example 24 was repeated using (5Z,7E)-(1R,2R,3R,24R)-1,3,24,25-tetrakis(t-butyldinethylsilyloxy)-2-(3-t-butyldimethylsllyloxypropoxy)-9,10-secocholesta-5,7,10(19)-triene (compound 20b; 3.3 mg, 3.06 µmol), toluene (0.5 ml), a 1M-TBAF-THF solution (153 µl, 237 µmol) to give the titled compound (compound 1b; 1.02 mg, 66%) as a colorless oil.
[a]²⁴_{D} -23° (c0.07, EtOH);
¹H NMR (270 MHz, CDCl₃) δ 0.56(s, 3H), 0.94(d, J=5.9Hz, 3H), 1.16(s, 3H), 1.22(s, 3H), 2.42(brd, J=14.5Hz, 1H), 2.55(dd, J=14.5, 4.0 Hz, 1H), 2.76-2.87(m, 1H), 3.27(dd, J=8.8, 2.8Hz, 1H), 3.30-3.37(m, 1H), 3.68-3.78(m, 1H), 3.80-4.00(m, 3H), 4.21-4.36(m, 2H), 5.08(brs, 1H), 5.50(brs, 1H), 6.04(d, J=11.2Hz, 1H), 6.36(d, J=11.2Hz, 1H);
IR(neat, cm⁻¹) 3400, 2947, 2927, 2871, 1377, 1105, 1070;
UV(EtOH)λ max 264 nm.

The reactions involved in the above illustrative Examples 24 and 25 are shown below.

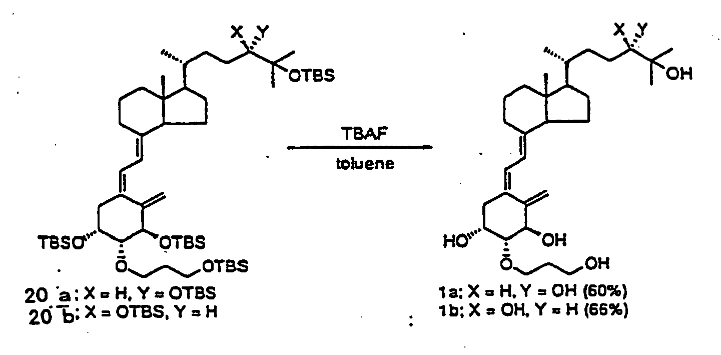

### Reference Example 3: Synthesis of [3a(R)]-4(S)-acetyloxy-1(R)-[1(S),5-dimethyl-5-triethylsilyloxyhexyl]-7a(R)-methyloctahydro-1H-indene (compound 2)

Triethylsilyl trifluoromethanesulfonate (TESOTf) (1.3 ml, 5.53 mmol) was added to a solution of [3a(R)]-4(S)-acetyloxy-1(R)-[1(S),5-dimethyl-5-hydroxyhexyl]-7a(R)-methyloctahydro-1H-indene (compound 1, Hatakeyama, S. et al. disclosed in The Journal of Organic Chemistry, 56, 461 (1991), 1.38 g, 4.25 mmol) and 2,6-lutidine (0.79 ml, 6.80 mmol) in dichloromethane (10 ml), followed by stirring for 30 min. The reaction solution was diluted with dichloromethane, washed with ice-cooled 0.5N hydrochloric acid and saturated aqueous sodium hydrogencarbonate and dried over magnesium sulfate. The dried product was distilled under reduced pressure to remove the solvent and purified by silica gel column chromatography (hexane:ethyl acetate = 19:1) to give the titled compound (1.66 g, 89%) as a colorless oil.
¹H NMR (200 MHz, CDCl₃), δ 0.49-0.63(m, 6H), 0.87-1.01(m, 15H), 1.18(s, 6H), 2.04(s, 3H), 5.14(br, 1H).

### Reference Example 4: Synthesis of [3a(R)]-1(R)-[1(S),5-dimethyl-5-triethylsilyloxyhexyl]-4(S)-hydroxy-7a(R)-methyloctahydro-1H-indene (compound 3)

A portion of [3a(R)]-4(S)-acetyloxy-1(R)-[1(S),5-dimethyl-5-triethylsilyloxyhexyl]-7a(R)-methyl-octahydro-1H-indene (compound 2, 1.65 g, 3.76 mmol) obtained in Reference Example 3 was dissolved in THF (15 ml). The solution was cooled to 0°C and then slowly mixed with LiAlH₄ (214 mg, 5.64 mmol), followed by stirring for 30 min. at room temperature. After treating the excess LiAlH₄ with ethyl acetate, the mixture was poured into a 10% aqueous NaOH solution and extracted twice with ethyl acetate. The extract was washed with brine, dried over anhydrous magnesium sulfate and then distilled under reduced pressure to remove the solvent. The resulting residue was purified by silica gel column chromatography (n-hexane: ethyl acetate = 10:1) to give the titled compound (1.57 g, quantitatively) as a colorless oil. ¹H NMR (200 MHz, CDCl₃), δ 0.49-0.63(m, 6H), 0.86-1.01(m, 15H), 1.18(s, 6H), 4.07(br, 1H).

The reactions involved in the above Reference Examples 3 and 4 are shown below.

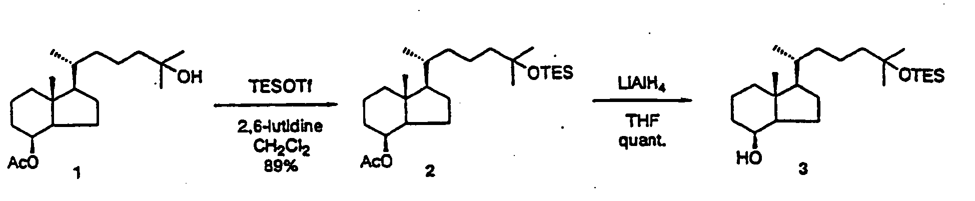

### Reference Example 5: Synthesis of [3a(R)]-1(R)-[1(S),5-dimethyl-5-triethylsilyloxyhexyl]-7a(R)-methyl-4-oxooctahydro-1H-indene

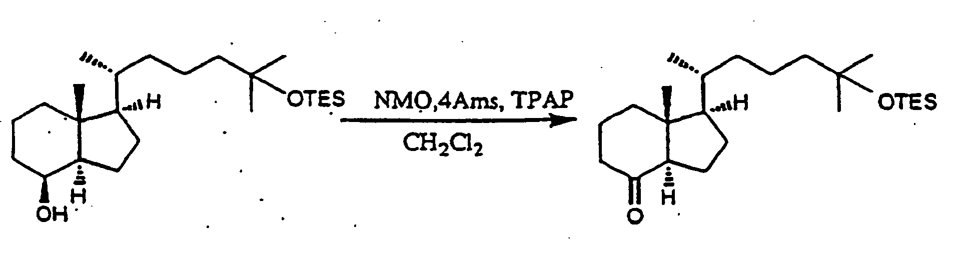

NMO (4.1 mg, 0.035 mmol) and 4A molecular sieve (12.9 mg) were added to a solution in dichloromethane (3 ml) of the [3a(R)]-1(R)-[1(S),5-dimethyl-5-triethylsilyloxyhexyl]-4(S)-hydroxy-7a(R)-methyl-octahydro-1H-indene (10.3 mg, 0.026 mmol) obtained in Reference Example 4, followed by stirring for 1 hour at room temperature under argon atmosphere. TPAP (0.5 mg, 0.0014 mmol) was further added to the mixture. After 6.5 hours, the reaction solution was filtered with Celite and the filtrate was concentrated under reduced pressure. The resulting residue was purified by column chromatography (n-hexane:ethyl acetate = 5:1) to give the titled compound (10.0 mg, 97%) as a colorless oil.
¹H NMR (300 MHz, CDCl₃) δ 2.45(dd, 1H, J=9.0Hz, 12.5Hz), 1.21-2.35(m, 18H), 1.19(s, 6H), 0.94(s, 3H), 0.94(t, 9H, J=8.0Hz), 0.65(s, 3H), 0.56(q, 6H, J=8.0Hz).

### Reference Example 6: Synthesis of [3a(R)]-4(E)-bromomethylene-1(R)-[1(S),5-dimethyl-5-triethylsilyloxyhexyl]-7a(R)-methyl-octahydro-1H-indene

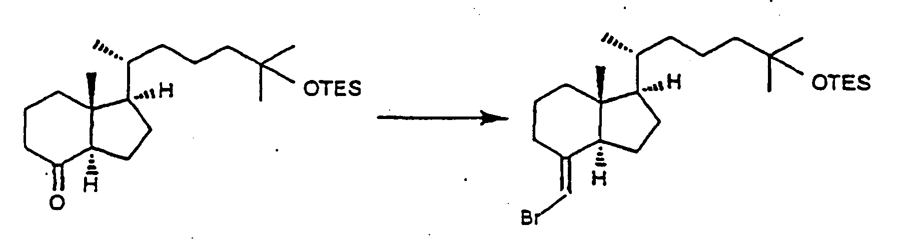

Sodium bis(trimethylsilyl)amide (in THF at 1M , 455 µl, 0.46 mmol) was added to a solution of (bromomethylene)triphenylphosphonium bromide (205 mg, 0.47 mmol) in THF (1.1 ml) at -60°C, followed by stirring under argon atmosphere for 1 hour. After a solution in THF (0.3 ml) of the [3a(R)]-1(R)-[1(S),5-dimethyl-5-triethylsilyloxyhexyl]-7a(R)-methyl-4-oxo-octahydro-1H-indene (23.1 mg, 0.059 mmol) obtained in Reference Example 5 was added to the mixture, the reaction temperature was immediately raised to room temperature. After 1 hour, the mixture was diluted with n-hexane and filtered with silica gel; the filtrate was concentrated under reduced pressure. The resulting residue was purified by preparative TLC (n-hexane) to give the titled compound (10.0 mg, 38%) as a yellow oil.
[α]_{D}²³ +62.1° (c0.33, CHCl₃);
IR (neat) 1462, 1235cm⁻¹;
¹H NMR (200MHz, CDCl₃) δ 5.64(br t, 1H, J=1.6Hz), 2.86(br dd, 1H, J=9.2, 4.0Hz), 1.20-2.10(m, 18H), 1.18(s, 6H), 0.94(t, 9H, J=7.9Hz), 0.90(s, 3H), 0.56(s, 3H), 0.56(q, 6H, J=7.9Hz).

### Example 26: Synthesis of (5Z,7E)-(1R,2R,3R)-1,3-bis(t-butyldimethylsilyloxy)-2-(3-t-butyldimethylsilyloxypropoxy)-25-triethylsilyloxy-9,10-secocholesta-5,7,10(19)-triene

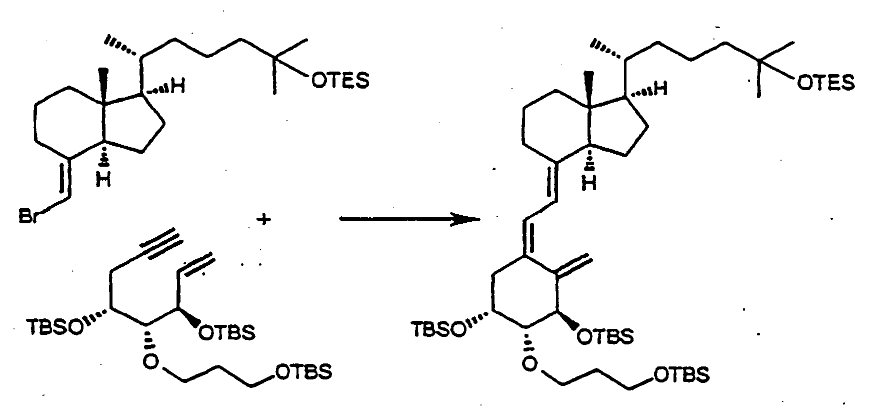

A mixture solution of triphenylphosphine (1.7 mg, 0.0065 mmol) and tris(dibenzylideneacetone)(chloroform)dipalladium(0) (0.9 mg, 0.00087 mmol) in toluene (0.2 ml) and triethylamine (0.3 ml) was stirred at room temperature for 10 min. and then mixed with a solution in toluene (0.2 ml) of the [3a(R)]-4(E)-bromomethylene-1(R)-[1(S),5-dimethyl-5-triethylsilyloxyhexyl]-7a(R)-methyl-octahydro-1H-indene (10.0 mg, 0.021 mmol) obtained in Reference Example 6 and the 3(R)-3,5-bis(t-butyldimethylsilyloxy)-4-(3-t-butyldimethylsilyloxypropoxy)-1-octen-7-ine (8.2 mg, 0.015 mmol) obtained in Example 21, followed by heating under reflux for 4.5 hours. The resultant was diluted with n-hexane and filtered with silica gel; the filtrate was concentrated under reduced pressure. The resulting residue was purified by preparative TLC (n-hexane:benzene = 2:1) to give the titled compound (3.5 mg, 26%) as a colorless oil.
¹H NMR (500MHz, CDCl₃) δ 6.22(d, 1H, J=11.2Hz), 6.00(d, 1H, J=11.2Hz), 5.26(s, 1H), 4.98(d, 1H, J=2.7Hz), 4.23(d, 1H, J=6.4Hz), 4.18(m, 1H), 3.61-3.74(m, 4H), 3.23(br d, 1H, J=6.4Hz), 2.81(d, 1H, J=11.8Hz), 2.46(dd, 1H, J=13.0Hz, 8.9Hz), 2.21(dd, 1H, J=13.0Hz, 3.4Hz), 1.19-2.09(m, 32H), 0.88-0.97(m, 36H), 0.51-0.59(m, 6H), 0.04-1.00(m, 18H).

## Claims

1. A process for preparing vitamin D derivatives having the general formula (6): 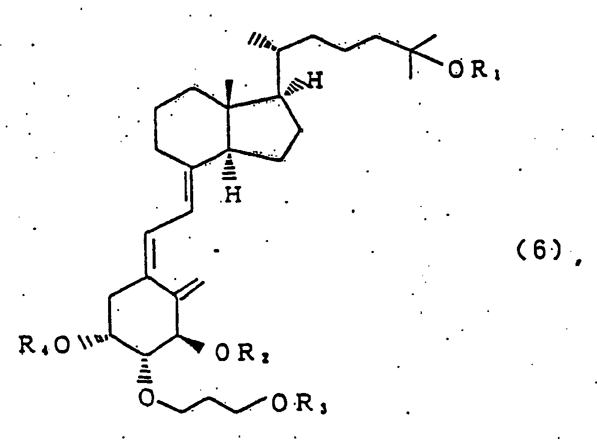 wherein R₁, R₂, R₃ and R₄, which may be the same or different, represent a hydrogen atom or a protecting group, comprising:
reacting a compound having the general formula (7): 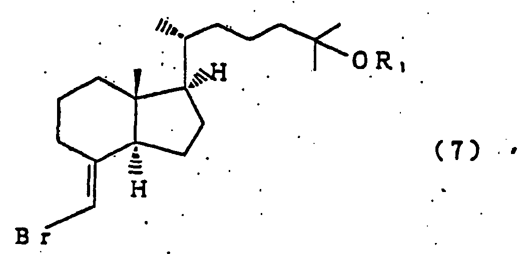 wherein R₁ represents a hydrogen atom or a protecting group,
with a compound having the general formula (5): 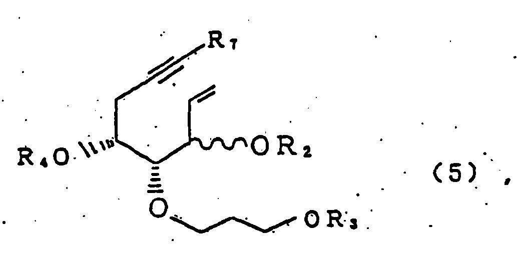 wherein R₇ represents a hydrogen atom; and R₂, R₃ and R₄, which are the same or different, represent a hydrogen atom or a protecting group.

## Patentansprüche

1. Verfahren zur Herstellung von Vitamin D-Derivaten der allgemeinen Formel (6): 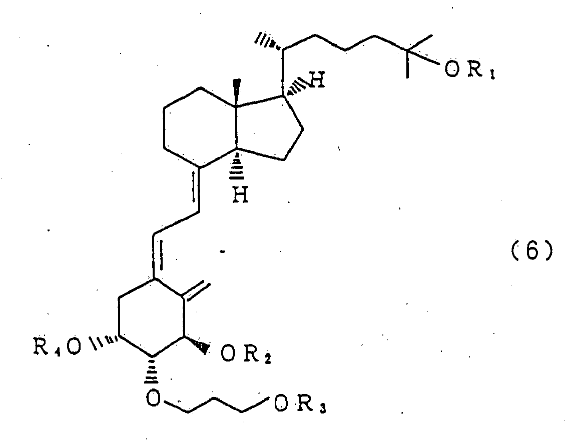 wobei R₁, R₂, R₃ und R₄, welche gleich oder verschieden sein können, ein Wasserstoffatom oder eine Schutzgruppe darstellen,
umfassend:
Umsetzen einer Verbindung der allgemeinen Formel (7): 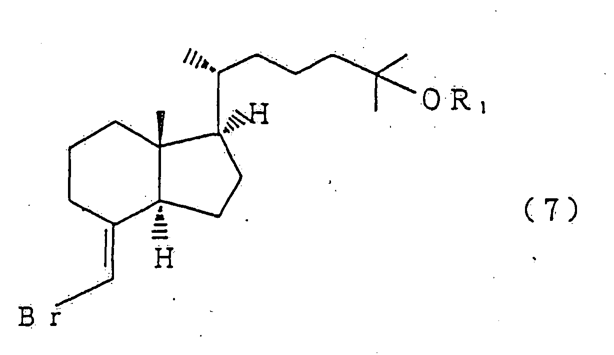
wobei R₁ ein Wasserstoffatom oder eine Schutzgruppe darstellt, mit einer Verbindung der allgemeinen Formel (5): 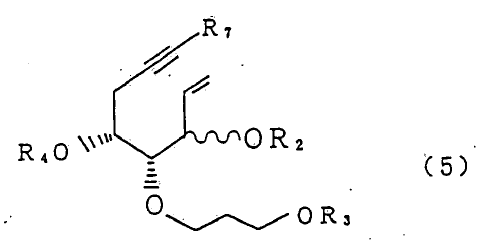 wobei R₇ ein Wasserstoffatom darstellt; und R₂, R₃ und R₄, welche gleich oder verschieden sind, ein Wasserstoffatom oder eine Schutzgruppe darstellen.

## Revendications

1. Un procédé pour la préparation de dérivés de vitamine D ayant la formule générale (6) suivante: 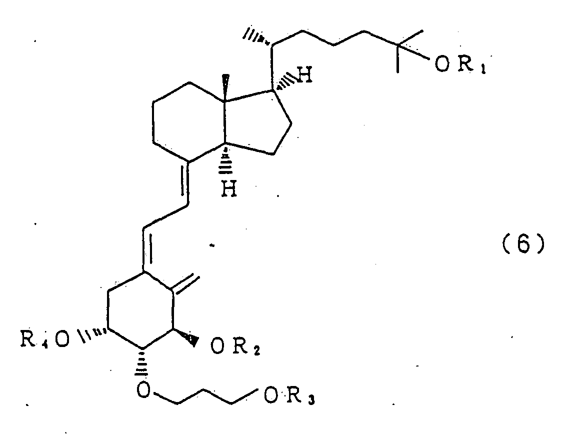 où R₁, R₂, R₃ et R₄, qui peuvent être identiques ou différents, représentent un atome d'hydrogène ou un groupe protecteur,
comprenant:
une mise en réaction d'un composé ayant la formule générale (7) suivante: 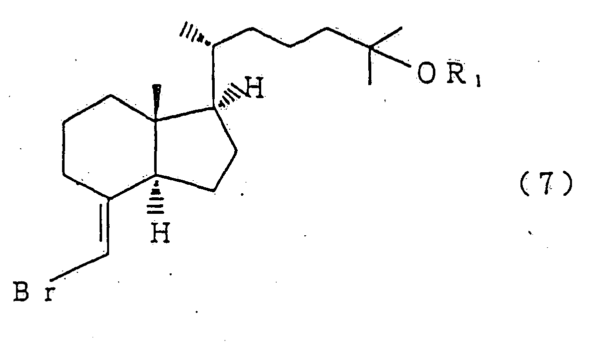
où R₁ représente un atome d'hydrogène ou un groupe protecteur,
avec un composé ayant la formule générale (5) suivante: 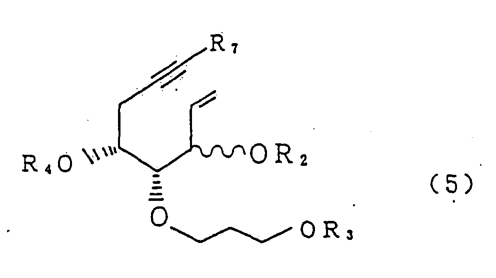 où R₇ représente un atome d'hydrogène; et R₂, R₃, et R₄, qui sont identiques ou différents, représentent un atome d'hydrogène ou un groupe protecteur.
